# EUROPÄISCHE PATENTSCHRIFT

(11) **EP 1 458 296 B1**
(45) Veröffentlichungstag und Bekanntmachung des Hinweises auf die Patenterteilung: **14.06.2006**
(21) Anmeldenummer: 02792896.9
(22) Anmeldetag: 05.12.2002
(51) Int. Cl.: A61B 17/132

(54) **ABSCHNÜRVORRICHTUNG FÜR KÖRPERTEILE**
LIGATURE DEVICE FOR BODY PARTS
DISPOSITIF DE LIGATURE POUR PARTIES DU CORPS

(30) Priorität: 15.12.2001 DE 10161749
(43) Veröffentlichungstag der Anmeldung: 22.09.2004
(73) Patentinhaber: KIMETEC GMBH MEDIZINTECHNIK, D-71254 Ditzingen (DE)
(72) Erfinder: KIRCHNER, Claudia, 71706 Markgröningen (DE)
(74) Vertreter: Fleck, Hermann-Joseph
(86) Internationale Anmeldenummer: PCT/EP2002/013773
(87) Internationale Veröffentlichungsnummer: WO 2003/051207

(56) Entgegenhaltungen:
- WO-A-88/00456
- DE-U- 9 418 598
- DE-U- 29 602 462

## Beschreibung

Die Erfindung bezieht sich auf eine Abschnürvorrichtung für Körperteile mit einem Schlossgehäuse, das eine Bodenwand, zwei Seitenwände und auf seiner Oberseite eine Deckwand aufweist, mit einem mit einem Ende am Schlossgehäuse mittels eines von der Rückseite her zwischen einer Zwischenwand und der Deckwand einführbaren Rastschuhs lösbar angekoppelten oder ankoppelbaren Abschnürband, das unter Bildung einer Schlaufe mit seinem anderen freien Ende zwischen einer über der Bodenwand schwenkbar gelagerten Wippe und einer unter Abstand darüber angeordneten Zwischenwand durch das Schlossgehäuse geführt oder führbar und mit der Wippe gegen einen vorderen, mit dem Schlossgehäuse verbundenen Abschnitt der Zwischenwand einklemmbar ist.

Eine derartige Abschnürvorrichtung ist in der DE 42 10 255 C1 und auch der DE 94 18 598 U1 angegeben. Oberhalb einer Bodenwand des Schlossgehäuses ist eine Wippe schwenkbar gelagert, die auf ihrer Vorderseite in einen konvex nach oben und hinten geführten Betätigungsabschnitt übergeht und in dem Übergangsbereich eine Durchführöffnung 4.3 zum Herausführen eines oberhalb der Wippe verlaufenden Abschnürbandes aufweist, das auf der Rückseite des Schlossgehäuses eine um das abzuschnürende Körperteil legbare Schlaufe bildet und mit einem Rastschuh in einen Aufnahmeschacht auf der Rückseite des Gehäuses eingeführt ist und verrastet ist. Mittels eines vorderen Abschnitts der Wippe wird das Abschnürband gegen die Unterseite einer zwischen der Bodenwand und einer Deckwand angeordneten Zwischenwand gedrückt und eingeklemmt, wenn der hintere Abschnitt der Wippe beim Abschnüren nach unten zu der Bodenwand hin gezogen wird. Der Rastschuh weist eine nach oben abstehende und auf der Unterseite der Deckwand sich abstützende Rastzunge auf, die mittels eines darüber angeordneten, separaten Druckteils zum Entrasten und Herausziehen des Rastschuhs mit dem betreffenden Abschnürbandende nach unten drückbar ist.

Der Erfindung liegt die Aufgabe zugrunde, eine Abschnürvorrichtung der genannten Art bereitzustellen, die Vorteile hinsichtlich des Aufbaus und der Handhabung bietet.

Diese Aufgabe wird mit den Merkmalen des Anspruchs 1 gelöst. Hiernach ist vorgesehen, dass die Zwischenwand einen zur Oberseite abstehenden federnden Abschnitt aufweist, mit dem der Rastschuh in seinem hinteren Bereich angehoben und im eingeführten Zustand mit seiner hinteren Oberseite gegen ein gehäusefestes Widerlager gedrückt ist, wobei der Rastschuh mit einem rückseitigen Halteelement und/oder einem vorderseitigen Halteteil unter Zusammenwirken mit einem Gegenelement oder Gegenstück des Schlossgehäuses gegen ein Herausziehen nach hinten gehalten und durch Niederdrücken seines hinteren Bereichs freigebbar ist.

Mit den Maßnahmen des Anspruchs 1 ist kein separates Druckteil erforderlich, insbesondere wird auch eine für eine sichere Funktion präzise ausgebildete Zunge in dem als bewegliches Teil ausgebildeten Rastschuh eingespart und eine äußerst einfache Handhabung erreicht.

Ist vorgesehen, dass der Rastschuh zum Einstecken in das Schlossgehäuse einen Steckabschnitt aufweist und zum Freigeben mit einem an dessen Hinterseite angeschlossenen Betätigungsabschnitt versehen ist, so kann der Rastschuh auf einfache Weise eingesteckt und gelöst werden, wobei der einfache Aufbau begünstigt wird. Zum Steuern der Schlaufenspannung über die Wippe einerseits und das vollständige Öffnen der Schlaufe über den Rastschuh andererseits sind lediglich zwei beweglich gelagerte Betätigungsteile erforderlich.

Die Bedienung wird dadurch erleichtert, dass der Betätigungsabschnitt auf seiner Oberseite mit einer Auslösemulde versehen ist und dass in ihm eine Fixieraufnahme zum Festlegen des Abschnürbandes ausgebildet ist, so dass sich die Bedienperson vollständig auf ihre eigentliche Aufgabe konzentrieren kann.

Eine vorteilhafte Ausbildung für den Aufbau besteht darin, dass die Zwischenwand mit ihrem vorderen Abschnitt beidseitig an der Innenseite der benachbarten Seitenwände angeformt ist, während ihr hinterer Abschnitt mit einem jeweiligen Spalt von den benachbarten Seitenwänden oder daran angeformten Stegen der Zwischenwand getrennt ist.

Zum Erzielen einer sicheren Verrastung sind weiterhin die Maßnahmen vorteilhaft, dass der Rastschuh auf der Unterseite seines vorderen Abschnitts als Halteteil eine nach unten überstehende Haltenase oder eine Vertiefung mit Halteabsatz aufweist, wobei die unterseitige Haltenase mit einer Haltekante oder die Vertiefung mit einem Haltevorsprung an der Oberseite des vorderen Abschnitts der Zwischenwand zusammenwirkt.

Für eine sichere Verrastung kann weiterhin vorteilhaft vorgesehen sein, dass das rückseitige Halteelement auf der Oberseite des Rastschuhs als nach oben abstehende Haltenase oder als Vertiefung ausgebildet ist, wobei die oberseitige Haltenase oder Vertiefung mit dem einen Stützabschnitt aufweisenden Gegenelement zusammenwirkt.

Eine sichere Wirkungsweise der Abschnürvorrichtung wird weiterhin dadurch unterstützt, dass die Zwischenwand zum Festklemmen des Abschnürbandes mittels der Wippe auf ihrer Unterseite am vorderen Rand eine nach unten vorgezogene, spitzwinklige Haltekante als Klemmstruktur aufweist, die gegen ein Zurückziehen des Abschnürbandes angestellt ist.

Die Funktion und Handhabung sowie ein einfacher Aufbau werden dadurch begünstigt, dass an der Vorderseite oder Wippe unter Freilassen einer Durchführöffnung für das Abschnürband ein nach hinten geführter, konvex nach oben gekrümmter Betätigungsabschnitt angeformt ist, der auf seiner Oberseite mit einer nach oben konkaven Betätigungsmulde versehen ist.

Eine weitere günstige Maßnahme, mit der z.B. eine einfache Zuordnung und damit die Bedienung begünstigt werden besteht darin, dass auf der Oberseite oder Deckwand eine Ausnehmung mit einem darin lösbar aufgenommenen Einsatzteil vorgesehen ist.

Dabei besteht eine für den Aufbau und die Handhabung vorteilhafte Ausgestaltung darin, dass die Ausnehmung seitliche, zur Rückseite offene Führungsnuten aufweist, in die das Einsatzteil einschiebbar ist, und dass die Kontur eines hinteren Randes des Einsatzteils im eingeschobenen Zustand mit der Kontur des hinteren Randes der Deckwand des Schlossgehäuses bündig ist und dass die Kontur der Vorderseite des Betätigungsabschnitts an die Kontur des hinteren Randes des Einsatzteils und der Deckwand angepasst ist.

Die Erfindung wird nachfolgend anhand eines Ausführungsbeispiels unter Bezugnahme auf die Zeichnungen näher erläutert. Es zeigen:
- Fig. 1: eine perspektivische Ansicht einer Abschnürvorrichtung,
- Fig. 2: eine perspektivische Ansicht eines Schlossgehäuses der Abschnürvorrichtung nach Fig. 1,

- Fig. 3a) bis e): verschiedene Ansichten des Schlossgehäuses nach Fig. 2,
- Fig. 4a) bis d): verschiedene Ansichten einer Wippe der Abschnürvorrichtung nach Fig. 1,
- Fig. 5a) bis d): verschiedene Ansichten eines Rastschuhs einer Abschnürvorrichtung nach Fig. 1,
- Fig. 6a) bis c): verschiedene Ansichten eines Einsatzteils bei einer Abschnürvorrichtung nach Fig. 1,
- Fig. 7a) bis d): verschiedene Ansichten eines Fixierteils für ein Abschnürband einer Abschnürvorrichtung und
- Fig. 8a) bis d): verschiedene Ansichten eines Gegenstücks zum Festlegen des Abschnürbandes mittels eines Fixierteils nach Fig. 7 an einem Rastschuh nach Fig. 5.

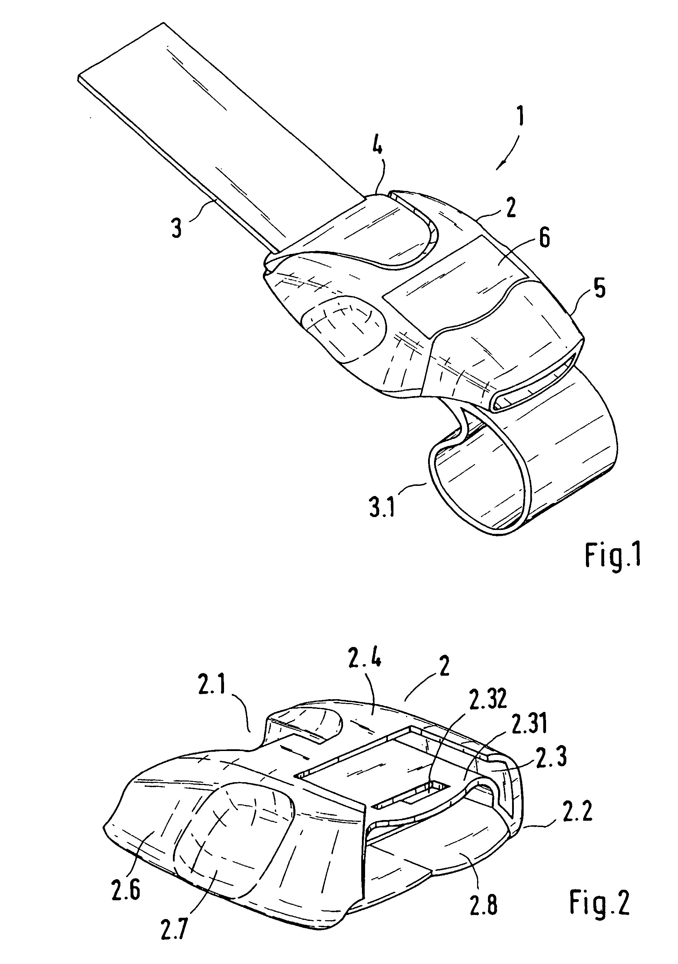

Eine in Fig. 1 gezeigte Abschnürvorrichtung 1 weist als wesentliche Bestandteile ein Schlossgehäuse 2, ein durch dieses geführtes, auf der Hinterseite des Schlossgehäuses 2 unter Bildung einer Schlaufe 3.1 mittels eines Rastschuhs 5 lösbar festgelegtes Abschnürband 3 sowie eine zum dosierbaren Einklemmen des Abschnürbands 3 im Schlossgehäuse 2 angeordnete Wippe 4 auf. Auf der Oberseite einer Deckwand 2.4 des Schlossgehäuses 2 ist in eine entsprechend ausgebildete Ausnehmung 2.3 ein Einsatzteil 6 z. B. als Informationsplakette eingesetzt, wie in Verbindung mit Fig. 2 ersichtlich.

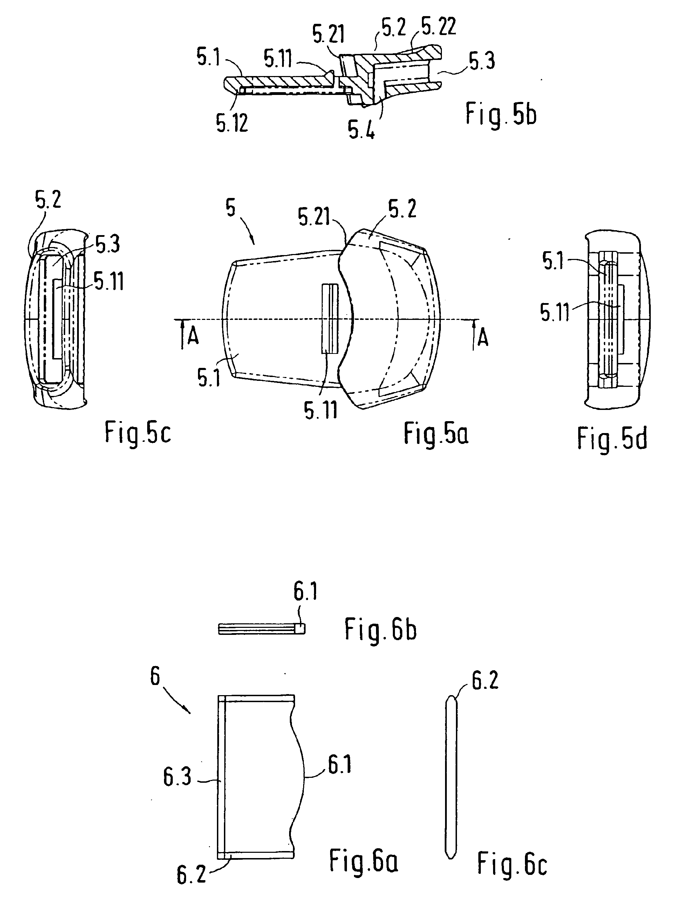

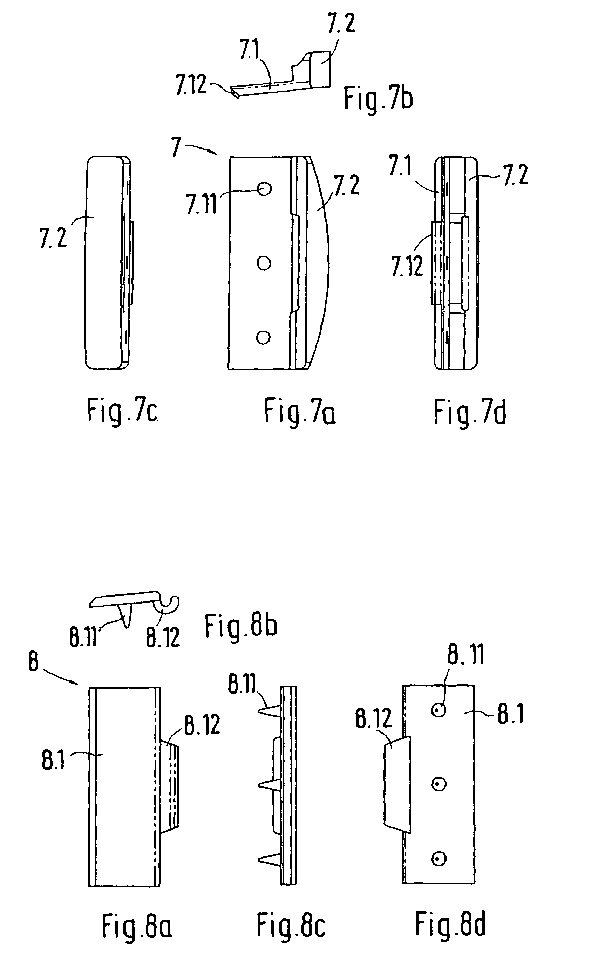

Das in Fig. 2 näher dargestellte Schlossgehäuse 2 ist von zwei Seitenwänden 2.6 mit jeweiligen Griffmulden 2.7, einer Bodenwand 2.8 und einer Deckwand 2.4 umgrenzt und weist auf seiner Rückseite eine Einführöffnung für das Abschnürband 3 und auch den Rastschuh 5 sowie das Einsatzteil 6 auf. Auf seiner Vorderseite ist das Schlossgehäuse 2 mit einer Ausführöffnung 2.1 versehen, in die die Wippe 4 einsetzbar und aus der das Abschnürband 3 mit seinem einen Ende herausgeführt ist. Die Ausnehmung 2.3 ist auf ihrer Unterseite mit einem Wandabschnitt 2.31 abgegrenzt, in der ein als rechteckiges Fenster ausgebildeter Halteabschnitt 2.32 vorgesehen ist, und weist seitliche Führungsnuten zum Einschieben des Einsatzteils 6 von der Rückseite 2.2 aus auf, wobei die Führungsnuten zwischen dem Wandabschnitt 2.31 und beiderseitigen Abschnitten der Deckwand 2.4 sowie den Nutgrund bildenden Abschnitten der Seitenwände 2.6 gebildet sind. Auch im vorderen Bereich der Ausnehmung 2.3 kann diese nutartig ausgebildet sein, um auch einen vorderen Rand 6.3 des Einsatzteils 6 (vgl. Fig. 6) im vollständig eingeschobenen Zustand aufzunehmen.

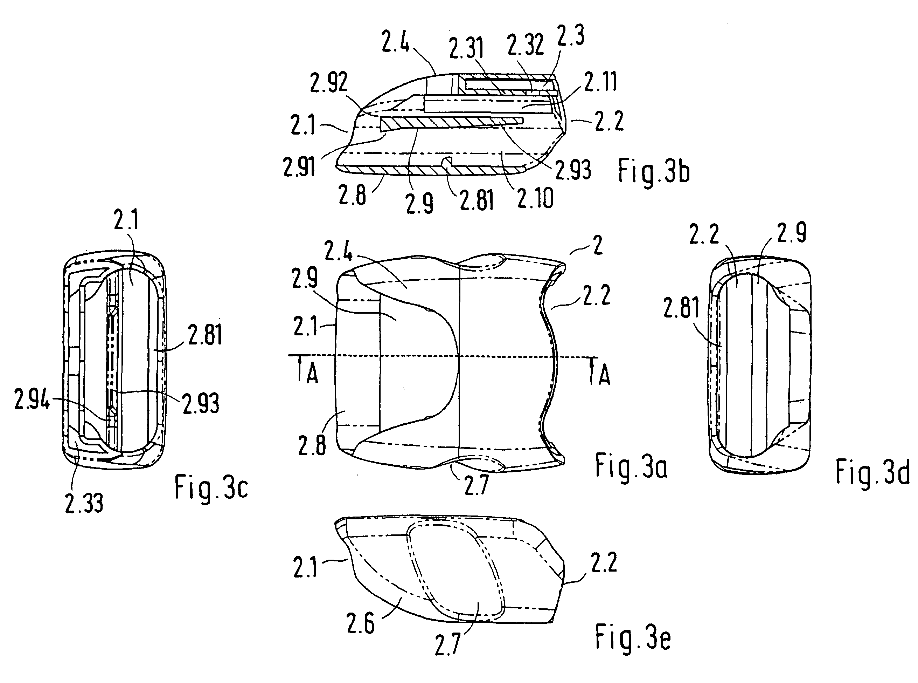

Wie aus den Ansichten der Fig. 3, insbesondere den Darstellungen in Fig. 3b), 3c) und 3d) näher ersichtlich, ist innerhalb des Schlossgehäuses 2 zwischen der Deckwand 2.4 und der Bodenwand 2.8 eine Zwischenwand 2.9 angebracht, vorzugsweise angeformt, mit der zwischen der Bodenwand 2.8 und der Unterseite der Zwischenwand 2.9 ein Durchführschacht 2.10 für das Abschnürband 3 und zwischen der Oberseite der Zwischenwand 2.9 und der Unterseite der Deckwand 2.4 bzw. des daran angeformten Wandabschnitts 2.31 ein Aufnahmeschacht 2.11 zum Einführen eines Steckabschnitts 5.1 des Rastschuhs 5 (vgl. Fig. 5) gebildet sind. Die Zwischenwand 2.9 weist auf ihrer Unterseite eine Haltestruktur zum Festklemmen des Abschnürbands 3 auf und ist insbesondere an ihrer vorderen Unterkante 2.91 spitzwinklig ausgebildet, wodurch sich eine gewisse Anstellung gegen die Zugrichtung des Abschnürbands 3 im Abschnürzustand ergibt. Die vordere Oberkante bildet eine Haltekante 2.92 als Haltenase für ein hakenförmiges Halteteil 5.12 auf der Unterseite des Steckabschnitts 5.1 des Rastschuhs 5. Wie aus Fig. 3c) ersichtlich, ist im hinteren Abschnitt der Zwischenwand 2.1, der sich bis über die Mitte nach vorne erstrecken kann, ein federnder Abschnitt 2.93 durch seitliche Einschnitte bzw. Spalte 2.94 gegenüber seitlichen, mit den Seitenwänden 2.6 verbundenen Abschnitten der Zwischenwand 2.9 gebildet. Im vorderen Abschnitt ist die Zwischenwand 2.9 durchgehend mit den Seitenwänden 2.6 verbunden, insbesondere angeformt.

Auf der Oberseite der Bodenwand 2.8 des Schlossgehäuses 2 ist eine rippenartige Schwenkstütze 2.81 als Schwenklagerung und zum Halten der Wippe 4 angeformt, wie die Fig. 3b), 3c) und 3d) ebenfalls erkennen lassen.

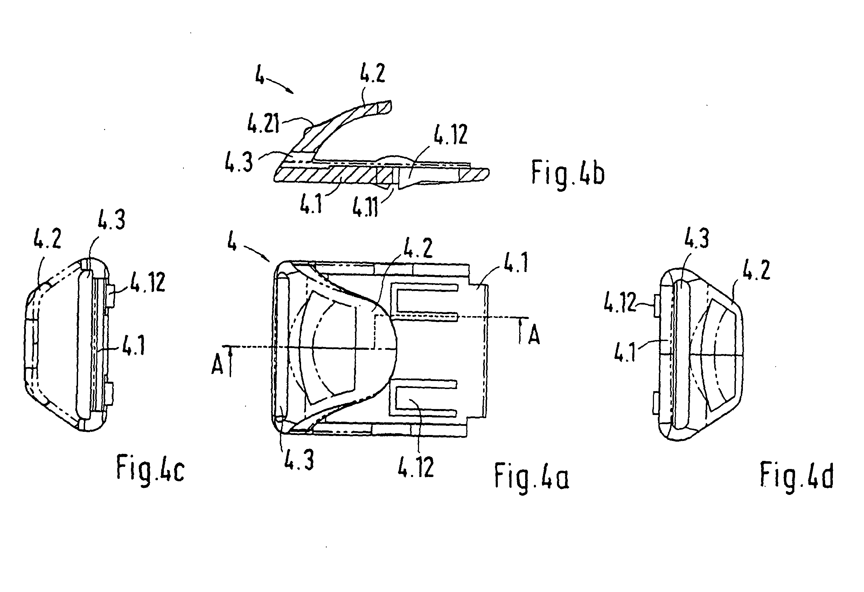

Einzelheiten der Wippe 4 sind in Fig. 4a) bis d) dargestellt. Die mit einem Schwenklager 4.11 zwischen quer verlaufenden Rippen oder Noppen auf der Schwenkstütze 2.81 schwenkbar gelagerte Wippenplatte 4.1 geht an ihrem vorderen Endbereich in einen nach vorne oben konvex ausgebildeten Betätigungsabschnitt 4.2 über, der etwa in seinem mittleren Bereich eine nach oben konkav geformte Betätigungsmulde 4.21 mit einem vorderen wulstigen Rand zum einfachen taktilen Erkennen und Betätigen aufweist. Im Übergangsbereich zwischen der Wippenplatte 4.1 und dem Betätigungsabschnitt 4.2 ist eine Durchführöffnung 4.3 für das Abschnürband 3 gebildet. Auf der Unterseite der Wippenplatte 4.1 ist das Schwenklager 4.11 auf seiner Rückseite mittels nach unten vorstehender Federzungen 4.12 mit einer nach hinten verlaufenden Einführschräge begrenzt, so dass die Wippe 4 von vorne leicht unter Führung in seitlichen Nuten oder ähnlichen Führungsbahnen in den Durchführschacht 2.10 des Schlossgehäuses 2 eingeführt und an der Schwenkstütze 2.81 verrastet und mittels der Federzungen 4.12 auch wieder freigegeben werden kann. Der Betätigungsabschnitt 4.2 ist in Draufsicht zungenartig und nach oben verjüngt in Form einer stetigen Krümmung ausgebildet und in eine entsprechend geformte Ausnehmung in der Deckwand 2.4 des Schlossgehäuses 2 eingepasst.

Der in Fig. 5a) bis d) näher gezeigte Rastschuh 5 weist in seinem hinteren Bereich einen an den Steckabschnitt 5.1 angeformten, im eingesetzten Zustand nach hinten über das Schlossgehäuse 2 herausragenden Betätigungsabschnitt 5.2 mit einer nach oben konkaven Auslösemulde 5.22 auf seiner Oberseite auf. Der Vorderrand des Betätigungsabschnitts 5.2 ist in Draufsicht symmetrisch gekrümmt mit zwei über einen mittleren Kurvenabschnitt stetig ineinander übergehenden seitlichen nach vorne konvexen Krümmungsabschnitten und an einen entsprechenden Konturverlauf des hinteren Randes der Deckwand 2.4 des Schlossgehäuses 2 angepasst, so dass sich im eingesetzten Zustand ein eindeutiger Sitz ergibt. Im Innern ist der Betätigungsabschnitt 5.2 mit einer nach der Rückseite offene Fixieraufnahme 5.3 zum Aufnehmen eines Fixierteils 7 mit Gegenstück 8 (vgl. Fig. 7 und 8) zum Festlegen des Abschnürbands 3 versehen, wobei das Abschnürband 3 durch eine Durchtrittsöffnung 5.4 auf der Unterseite des Betätigungsabschnitts 5.2 herausgeführt ist. Der an den Betätigungsabschnitt 5.2 angeformte, nach vorne anschließende Steckabschnitt 5.1 ist in seinem hinteren Randbereich mit einem nach oben ragenden Halteelement 5.11 versehen, mit dem sich der Rastschuh 5 im eingesetzten Zustand an dem hinteren Rand des Halteabschnitts 2.32 in dem Wandabschnitt 2.31 des Schlossgehäuses 2 abstützt, wobei der Rastschuh 5 mittels des hinteren, federnd wirkenden Abschnitts der Zwischenwand 2.9 nach oben gedrückt ist. Zusätzlich hakt der bereits erwähnte Halteteil 5.12 auf der Unterseite des vorderen Abschnitts des Steckabschnitts 5.1 an der vorderen Haltekante 2.92 der Zwischenwand 2.9 ein. Zum Lösen des Rastschuhs 5 braucht dieser lediglich durch Druck auf den Betätigungsabschnitt 5.2 nach unten gedrückt zu werden, wodurch das Halteelement 5.11 einerseits und das Halteteil 5.12 andererseits gelöst werden und der Rastschuh 5 entrastet und durch den Zug des vorzugsweise elastischen Abschnürbands 3 von selbst aus dem Aufnahmeschacht 2.11 zumindest teilweise herausgezogen wird und dann leicht vollständig entnommen werden kann, um die Schlaufe 3.1 zu öffnen.

Das in Fig. 6 näher dargestellte Einsatzteil 6 weist vorzugsweise im Querschnitt konische seitliche Ränder 6.2 und einen komplementär zu dem Vorderrand des Betätigungsabschnitts 5.2 des Rastschuhs verlaufenden hinteren Rand 6.2 auf und ist mit einer gewissen Klemmwirkung, aber von Hand herausschiebbar in der Ausnehmung 2.3 gehalten. Das Einsatzteil 6 kann zur Kenntlichmachung in einer gewünschten Farbe oder transparent zum Unterlegen mit einer Information, z.B. einem Namensschild, ausgebildet sein, oder selbst eine geeignete Aufschrift tragen.

Das in Fig. 7a) bis d) gezeigte Fixierteil weist eine Fixierplatte 7.1 mit Fixierelementen 7.11 in Form von Durchbrüchen und ein auf seiner Vorderseite nach unten abstehendes Hakenteil 7.12 auf und geht auf seiner Rückseite in einen angeformten Fixierblock 7.2 über. Zum Festlegen des Abschnürbandes 3. wird dieses mit einem Endbereich auf der Oberseite der Fixierplatte 7.1 aufgelegt, mittels des in Fig. 8 dargestellten Gegenstücks 8 mit einer Gegenplatte 8.1 abgedeckt und mittels Gegenelemente 8.11 in Form von auf die Durchbrüche 7.11 abgestimmten dornartigen Zapfen festgelegt, wonach der Fixierteil 7 und das Gegenstück 8 in die Fixieraufnahme 5.3 des Rastschuhs 5 eingeklemmt und darin mittels des Hakenteils 7.12 gehalten werden, wobei die Gegenplatte 8.1 sich mit einem Stützglied 8.12 in der Fixieraufnahme 5.3 gegenüber dem Hakenteil 7.12 abstützt, das in die Durchtrittsöffnung 5.4 eingreift.

Mit den beschriebenen Maßnahmen ergibt sich bei einfachem Aufbau bei guter Funktion eine einfache Bedienung.

## Patentansprüche

1. Abschnürvorrichtung (1) für Körperteile mit einem Schlossgehäuse (2), das eine Bodenwand (2.8), zwei Seitenwände (2.6) und auf seiner Oberseite eine Deckwand (2.4) aufweist, mit einem mit einem Ende am Schlossgehäuse (2) mittels eines von der Rückseite her zwischen einer Zwischenwand (2.9) und der Deckwand (2.4) einführbaren Rastschuhs (5) lösbar angekoppelten oder ankoppelbaren Abschnürband (3), das unter Bildung einer Schlaufe (3.1) mit seinem anderen freien Ende zwischen einer über der Bodenwand (2.8) schwenkbar gelagerten Wippe (4) und der unter Abstand darüber angeordneten Zwischenwand (2.9) durch das Schlossgehäuse (2) geführt oder führbar und mit der Wippe (4) gegen einen vorderen, mit dem Schlossgehäuse (2) verbundenen Abschnitt der Zwischenwand (2.9) einklemmbar ist,
**dadurch gekennzeichnet,**
**dass** die Zwischenwand (2.9) einen zur Oberseite abstehenden federnden Abschnitt (2.93) aufweist, mit dem der Rastschuh (5) in seinem hinteren Bereich angehoben und im eingeführten Zustand mit seiner hinteren Oberseite gegen ein gehäusefestes Widerlager (2.31) gedrückt ist, wobei der Rastschuh (5) mit einem rückseitigen Halteelement (5.11) und/oder einem vorderseitigen Halteteil (5.12) unter Zusammenwirken mit einem Gegenelement (2.32) oder Gegenstück (2.92) des Schlossgehäuses (2) gegen ein Herausziehen nach hinten gehalten und durch Niederdrücken seines hinteren Bereichs freigebbar ist.

2. Abschnürvorrichtung nach Anspruch 1,
**dadurch gekennzeichnet,**
**dass** der Rastschuh (5) zum Einstecken in das Schlossgehäuse (2) einen Steckabschnitt (5.1) aufweist und zum Freigeben mit einem an dessen Hinterseite angeschlossenen Betätigungsabschnitt (5.2) versehen ist.

3. Abschnürvorrichtung nach Anspruch 2,
**dadurch gekennzeichnet,**
**dass** der Betätigungsabschnitt (5.2) auf seiner Oberseite mit einer Auslösemulde (5.22) versehen ist und
**dass** in ihm eine Fixieraufnahme (5.3) zum Festlegen des Abschnürbandes (3) ausgebildet ist.

4. Abschnürvorrichtung nach einem der vorhergehenden Ansprüche,
**dadurch gekennzeichnet,**
**dass** die Zwischenwand (2.9) mit ihrem vorderen Abschnitt beidseitig an der Innenseite der benachbarten Seitenwände (2.6) angeformt ist, während ihr hinterer Abschnitt mit einem jeweiligen Spalt (2.94) von den benachbarten Seitenwänden (2.6) oder daran angeformten Stegen der Zwischenwand (2.9) getrennt ist.

5. Abschnürvorrichtung nach einem der vorhergehenden Ansprüche,
**dadurch gekennzeichnet,**
**dass** der Rastschuh (5) auf der Unterseite seines vorderen Abschnitts als Halteteil (5.12) eine nach unten überstehende Haltenase oder eine Vertiefung mit Halteabsatz aufweist, wobei die unterseitige Haltenase mit einer Haltekante (2.92) oder die Vertiefung mit einem Haltevorsprung an der Oberseite des vorderen Abschnitts der Zwischenwand zusammenwirkt.

6. Abschnürvorrichtung nach einem der vorhergehenden Ansprüche,
**dadurch gekennzeichnet,**
**dass** das rückseitige Halteelement (5.11) auf der Oberseite des Rastschuhs (5) als nach oben abstehende Haltenase oder als Vertiefung ausgebildet ist, wobei die oberseitige Haltenase oder Vertiefung mit dem einen Stützabschnitt aufweisenden Gegenelement zusammenwirkt.

7. Abschnürvorrichtung nach einem der vorhergehenden Ansprüche,
**dadurch gekennzeichnet,**
**dass** die Zwischenwand (2.9) zum Festklemmen des Abschnürbandes mittels der Wippe (4) auf ihrer Unterseite am vorderen Rand eine nach unten vorgezogene, spitzwinklige Haltekante als Klemmstruktur (2.91) aufweist, die gegen ein Zurückziehen des Abschnürbandes (3) angestellt ist.

8. Abschnürvorrichtung nach einem der vorhergehenden Ansprüche,
**dadurch gekennzeichnet,**
**dass** an der Vorderseite oder Wippe (4) unter Freilassen einer Durchführöffnung (4.3) für das Abschnürband (3) ein nach hinten geführter, konvex nach oben gekrümmter Betätigungsabschnitt (4.2) angeformt ist, der auf seiner Oberseite mit einer nach oben konkaven Betätigungsmulde (4.21) versehen ist.

9. Abschnürvorrichtung nach einem der vorhergehenden Ansprüche,
**dadurch gekennzeichnet,**
**dass** auf der Oberseite oder Deckwand (2.4) eine Ausnehmung (2.3) mit einem darin lösbar aufgenommenen Einsatzteil (6) vorgesehen ist.

10. Abschnürvorrichtung nach Anspruch 9,
**dadurch gekennzeichnet,**
**dass** die Ausnehmung (2.3) seitliche, zur Rückseite offene Führungsnuten (2.33) aufweist, in die das Einsatzteil (6) einschiebbar ist,
**dass** die Kontur eines hinteren Randes des Einsatzteils (6) im eingeschobenen Zustand mit der Kontur des hinteren Randes der Deckwand (2.4) des Schlossgehäuses (2) bündig ist und
**dass** die Kontur der Vorderseite des Betätigungsabschnitts (5.2) an die Kontur des hinteren Randes des Einsatzteils (6) und der Deckwand (2.4) angepasst ist.

## Claims

1. Ligature device (1) for body parts having a closure housing (2), which includes a base wall (2.8), two lateral walls (2.6) and a cover wall (2.4) on its upper side, and having a ligature strip (3), which is detachably coupled or couplable with one end to the closure housing (2) by means of a locking jaw (5), which is introducible from the rear between an intermediate wall (2.9) and the cover wall (2.4), said ligature strip (3) being guided or guidable with its other free end between a rocker (4), which is pivotably mounted above the base wall (2.8), and the intermediate wall (2.9), which is disposed at a spacing above the rocker (4), through the closure housing (2) forming a loop (3.1) and is clampable with the rocker (4) against a front section of the intermediate wall (2.9), which front section is connected to the closure housing (2), **characterised in that** the intermediate wall (2.9) includes a resilient portion (2.93), which stands out toward the upper side, and by means of which the locking jaw (5) is raised in its rear region and is pressed in the introduced state with its rear upper side against a support (2.31), which is fixed to the housing, wherein the locking jaw (5) is retained in opposition to being pulled out in a rearwards manner by means of a rear retaining member (5.11) and/or a front retaining part (5.12) interacting with a counter member (2.32) or counterpart (2.92) of the closure housing (2) and is releasable by pressing down on its rear region.

2. Ligature device according to claim 1, **characterised in that** for insertion into the closure housing (2) the locking jaw (5) includes a plug-in portion (5.1) and for release is provided with an actuating portion (5.2) which is connected to its rear.

3. Ligature device according to claim 2, **characterised in that** the actuating portion (5.2) is provided on its upper side with a release depression (5.22) and **in that** a fixing receiving means (5.3) is configured therein for securing the ligature strip (3).

4. Ligature device according to one of the preceding claims, **characterised in that** the intermediate wall (2.9) is integrally formed with its front portion on both sides on the inside of the adjacent lateral walls (2.6), whilst its rear portion is separated by a respective gap (2.94) from the adjacent lateral walls (2.6) or from the webs of the intermediate wall (2.9) integrally formed thereon.

5. Ligature device according to one of the preceding claims, **characterised in that** the locking jaw (5), on the underside of its front portion, includes as retaining part (5.12), a retaining nose, which protrudes downwards, or a recess with retaining projection, wherein the retaining nose on the underside interacts with a retaining edge (2.92) or the recess interacts with a retaining projection on the upper side of the front portion of the intermediate wall.

6. Ligature device according to one of the preceding claims, **characterised in that** the rear retaining member (5.11) on the upper side of the locking jaw (5) is in the form of an upwardly protruding retaining nose or in the form of a recess, wherein the upper retaining nose or recess interacts with the counter member which includes a support portion.

7. Ligature device according to one of the preceding claims, **characterised in that**, for clamping the ligature strip by means of the rocker (4), the intermediate wall (2.9) includes on its underside on the front edge an acute-angled retaining edge, pulled out downwards, as the clamping structure (2.91), which is set in opposition to the ligature strip being pulled back.

8. Ligature device according to one of the preceding claims, **characterised in that** an actuating portion (4.2), which is guided rearwards and is curved upwards in a convex manner, is integrally formed on the front or rocker (4) leaving free a lead-in opening (4.3) for the ligature strip (3), which actuating portion (4.2) is provided on its upper side with an upwardly concave actuating depression (4.21).

9. Ligature device according to one of the preceding claims, **characterised in that** on the upper side or cover wall (2.4) a recess (2.3) is provided with an insertion part (6), which is received therein in a detachable manner.

10. Ligature device according to claim 9, **characterised in that** the recess (2.3) includes lateral guide grooves (2.33) which are open to the rear and into which the insertion part (6) is insertable, **in that** the outline of a rear edge of the insertion part (6) in the inserted state is flush with the outline of the rear edge of the cover wall (2.4) of the closure housing (2) and **in that** the outline of the front of the actuating portion (5.2) is adapted to the outline of the rear edge of the insertion part (6) and of the cover wall (2.4).

## Revendications

1. Dispositif de ligature (1) pour parties corporelles, comprenant un boîtier de serrure (2) comportant une paroi de fond (2.8), deux parois latérales (2.6) et une paroi de recouvrement (2.4) à sa face supérieure, et un ruban de ligature (3) qui est ou peut être accouplé amoviblement audit boîtier de serrure (2) par une extrémité, au moyen d'un sabot encliquetable (5) pouvant être inséré entre une paroi intermédiaire (2.9) et la paroi de recouvrement (2.4), à partir de la face postérieure ; qui est ou peut être guidé à travers le boîtier de serrure (2) par son autre extrémité libre, en formant une boucle (3.1), entre une pièce basculante (4) montée à pivotement au-dessus de la paroi de fond (2.8) et la paroi intermédiaire (2.9) occupant, à distance, une position sus-jacente ; et qui peut être coincé contre un tronçon antérieur de ladite paroi intermédiaire (2.9), relié audit boîtier de serrure (2),
**caractérisé par le fait**
**que** la paroi intermédiaire (2.9) présente un tronçon élastique (2.93) faisant saillie vis-à-vis de la face supérieure, par lequel le sabot encliquetable (5) est soulevé dans sa région postérieure et est poussé à l'état inséré, par sa face supérieure postérieure, contre une pièce d'arrêt (2.31) faisant corps avec le boîtier, sachant que ledit sabot encliquetable (5) est empêché d'être extrait vers l'arrière par un élément postérieur de retenue (5.11) et/ou par une pièce antérieure de retenue (5.12), en coopérant avec un élément complémentaire (2.32) ou avec une pièce complémentaire (2.92) du boîtier de serrure (2), et peut être libéré par enfoncement de sa région postérieure.

2. Dispositif de ligature selon la revendication 1,
**caractérisé par le fait**
**que** le sabot encliquetable (5) présente une zone d'encliquetage (5.1) en vue de l'emboîtement dans le boîtier de serrure (2) et est muni, en vue de la libération, d'une zone d'actionnement (5.2) se rattachant à sa face postérieure.

3. Dispositif de ligature selon la revendication 2,
**caractérisé par le fait**
**que** la zone d'actionnement (5.2) est pourvue d'une cuvette de déclenchement (5.22) sur sa face supérieure ; et
par le fait qu'un logement de verrouillage (5.3) est ménagé, dans ladite zone, en vue du blocage à demeure du ruban de ligature (3).

4. Dispositif de ligature selon l'une des revendications précédentes,
**caractérisé par le fait**
**que** la paroi intermédiaire (2.9) fait corps de part et d'autre, par son tronçon antérieur, avec la face interne des parois latérales (2.6) voisines, tandis que son tronçon postérieur est séparé, par un interstice respectif (2.94), d'avec lesdites parois latérales (2.6) voisines ou d'avec des membrures de la paroi intermédiaire (2.9) qui sont solidaires desdites parois latérales.

5. Dispositif de ligature selon l'une des revendications précédentes,
**caractérisé par le fait**
**que** le sabot encliquetable (5) comporte sur la face inférieure de son tronçon antérieur, en tant que pièce de retenue (5.12), un bec de retenue saillant vers le bas ou un renfoncement doté d'un mentonnet de retenue, ledit bec inférieur de retenue coopérant avec une arête de retenue (2.92), ou ledit renfoncement coopérant avec une protubérance de retenue à la face supérieure du tronçon antérieur de la paroi intermédiaire.

6. Dispositif de ligature selon l'une des revendications précédentes,
**caractérisé par le fait**
**que** l'élément postérieur de retenue (5.11) est réalisé, à la face supérieure du sabot encliquetable (5), sous la forme d'un renfoncement ou d'un bec de retenue saillant vers le haut, ledit renfoncement ou ledit bec supérieur de retenue coopérant avec l'élément complémentaire qui présente une zone d'appui.

7. Dispositif de ligature selon l'une des revendications précédentes,
**caractérisé par le fait**
**que** la paroi intermédiaire (2.9) présente à sa face inférieure, sur le bord antérieur, en vue de coincer fermement le ruban de ligature au moyen de la pièce basculante (4), une arête de retenue à angle aigu, en débord vers le bas, matérialisant une structure de coincement (2.91) agencée pour interdire un retrait dudit ruban de ligature (3).

8. Dispositif de ligature selon l'une des revendications précédentes,
**caractérisé par le fait**
**qu'**une zone d'actionnement (4.2) à courbure convexe vers le haut, s'étendant vers l'arrière, est façonnée d'un seul tenant sur la face antérieure ou sur la pièce basculante (4), en réservant un orifice de passage (4.3) destiné au ruban de ligature (3) ; et est dotée, sur sa face supérieure, d'une cuvette d'actionnement (4.21) de forme concave vers le haut.

9. Dispositif de ligature selon l'une des revendications précédentes,
**caractérisé par le fait**
**qu'**un évidement (2.3), dans lequel une pièce intégrée (6) est logée amoviblement, est prévu sur la face supérieure ou sur la paroi de recouvrement (2.4).

10. Dispositif de ligature selon la revendication 9,
**caractérisé par le fait**
**que** l'évidement (2.3) présente des rainures latérales de guidage (2.33) ouvertes vers la face postérieure, dans lesquelles la pièce intégrée (6) peut être insérée ;
par le fait que le profil d'un bord postérieur de la pièce intégrée (6) se trouve, à l'état inséré, dans l'affleurement du profil du bord postérieur de la paroi de recouvrement (2.4) du boîtier de serrure (2) ; et
par le fait que le profil de la face antérieure de la zone d'actionnement (5.2) est adapté au profil du bord postérieur de ladite pièce intégrée (6) et de ladite paroi de recouvrement (2.4).
